(19) **Europäisches Patentamt**
**European Patent Office**
**Office européen des brevets**

(11) **EP 2 339 539 A1**

(12) **EUROPÄISCHE PATENTANMELDUNG**

(43) Veröffentlichungstag:
    **29.06.2011 Patentblatt 2011/26**

(51) Int Cl.:
    ***G06T 11/00*** (2006.01)

(21) Anmeldenummer: **10014671.1**

(22) Anmeldetag: **16.11.2010**

(84) Benannte Vertragsstaaten:
    **AL AT BE BG CH CY CZ DE DK EE ES FI FR GB GR HR HU IE IS IT LI LT LU LV MC MK MT NL NO PL PT RO RS SE SI SK SM TR**
    Benannte Erstreckungsstaaten:
    **BA ME**

(30) Priorität: **17.11.2009 DE 102009053664**

(71) Anmelder: **Ziehm Imaging GmbH**
    **90451 Nürnberg (DE)**

(72) Erfinder:
    • **Hoerndler, Klaus**
      **90482 Nuernberg (DE)**
    • **Fleischmann, Christof**
      **91096 Moehrendorf (DE)**
    • **Kachelriess, Marc, Prof. Dr.**
      **90419 Nuernberg (DE)**
    • **Ritschl, Ludwig**
      **91054 Erlangen (DE)**

(54) **Verfahren zur Korrektur von Strahlungsaufhärtungs- und Streustrahleneffekten in der Radiografie und Röntgen-Computertomografie**

(57)    Die Erfindung betrifft ein Verfahren zur empirischen Bestimmung einer Korrekturfunktion zur Korrektur von Strahlungsaufhärtungs- und Streustrahleneffekten von wasseräquivalentem Gewebe in der Projektionsradiografie und in der Computertomografie unter Verwendung eines bildgebenden Detektors. Die Korrektur erfolgt auf den aus den logarithmierten Detektorsignalen gewonnen Projektionswerten, wobei die korrigierten Projektionswerte durch eine von der bei der Röntgenprojektionsaufnahme angelegten Röhrenspannung abhängigen Korrekturfunktion dargestellt werden, deren Koeffizienten aus einem einzigen Kalibrierscan an einem objektähnlichen Kalibrierphantom aus wasserähnlichem Material ermittelt werden.

Fig. 4

**EP 2 339 539 A1**

**Beschreibung**

Die Erfindung betrifft ein Verfahren zur empirischen Bestimmung einer Korrekturfunktion zur Korrektur von Strahlungsaufhärtungs- und Streustrahleneffekten in der Projektionsradiografie und in der Computertomografie unter Verwendung eines bildgebenden Detektors.

Es ist bekannt, daß in der Projektionsradiografie und in der Computertomografie mit einem bildgebenden Detektor durch die Streustrahlung und durch die von der Röntgenröhrenspannung und von der Primärfilterung abhängigen spektralen Strahlaufhärtung ganz ähnliche Bildauswirkungen ausgehen. Obwohl die physikalischen Grundlagen der Effekte völlig anderer Art sind, können die Bildwirkungen des sogenannten Cuppings beider Ursachen in der Praxis nicht von einander unterschieden werden.

Die Erfindung geht davon aus, den Streustrahlungseffekt und den Effekt der Strahlaufhärtung durch eine einzige von der Röhrenspannung abhängigen Korrekturfunktion zu berücksichtigen, die auf den aus den logarithmierten Detektorsignalen gewonnen Projektionswerten aufsetzt, wobei die Koeffizienten der Korrekturfunktion aus einem einzigen Kalibrierscan mit variabler Röhrenspannung an einem objektähnlichen Kalibrierphantom aus wasserähnlichem Material ermittelt werden. Die empirisch bestimmten Koeffizienten werden unter der Annahme ermittelt, daß die Rekonstruktion eines elliptischen Zylinderphantoms aus wasserähnlichem Material aus den bei unterschiedlichen Röhrenspannungen gewonnenen Projektionsaufnahmen eines Scans homogen ist.

Die Aufhärtung der Röntgenstrahlung bei der Durchdringung eines absorbierenden Objektes bewirkt, daß in rekonstruierten Axialbildern die Bildelemente zur Bildmitte hin mit geringer werdenden Grauwerten rekonstruiert werden. Dieser so genannte Schüsseleffekt ("Cupping") verhindert einen homogenen Bildeindruck. Der Schüsseleffekt wird vermieden, wenn die Projektionsdaten auf eine gedachte monoenergetische Röntgenstrahlung umgerechnet werden. Diese Umrechnung erfolgt für Weichteilgewebe in einem prärekonstruktiven Schritt mit anschließender Bildrekonstruktion.

Im Gegensatz zur Radiographie führt Streustrahlung bei CT-Rekonstruktion nicht nur zu einer Verschlechterung des Signal/Rausch-Verhältnisses, sondern zusätzlich zu objektabhängigen Grauwertverfälschungen, wie Cupping sowie Balken- oder Schattenartefakte, die sowohl die quantitative Genauigkeit als auch die Erkennbarkeit von niedrigen Kontrasten stark beeinträchtigen können.

Aus der DE102005028216A1 ist ein zweistufiges Verfahren zur polychromatischen Strahlungsaufhärtungs- und Streustrahlenkorrektur in Röntgenprojektionsaufnahmen eines Scans bekannt, wobei in einem ersten Verfahrensschritt vorgesehen ist, eine Wasservorkorrektur auf den aus den logarithmierten Detektormeßwerten errechneten Projektionswerten vorzunehmen, wobei jeder Projektionswert additiv oder Multiplikation mit einem abgespeicherten Korrekturwert korrigiert wird.

Aus der deutschen Patentschrift DE102005053498B4 ist ein Verfahren zur Beschleunigung der Streustrahlenkorrektur bekannt, bei der die aus den logarithmierten Schwächungsdaten gewonnenen Projektionswerte mit einem Strahlungsaufhärtungsterm korrigiert werden und die Streustrahlungskorrektur als Verstärkungsfaktor für den Strahlungsaufhärtungskorrekturterm berücksichtigt wird.

Aus der deutschen Offenlegungsschrift DE102006040852A1 ist ein Verfahren zur multiplikativen Streustrahlungskorrektur bei der Röntgenbildgebung bekannt, bei dem von den logarithmierten Meßsignalen des Röntgendetektors Korrekturwerte subtrahiert werden, die aus einer Reihenentwicklung eines Logarithmus erhalten werden.

Aus der deutschen Offenlegungsschrift DE102006021373A1 ist bekannt, neben anderen Vorkorrekturen eine kombinierte Streustrahlen-und Aufhärtungskorrektur vorzunehmen.

Aus der deutschen Patentschrift DE19523090C1 ist ein Verfahren zur Streustrahlenkorrektur bekannt, bei der die logarithmierten Meßwerte mit einem Korrekturfaktor multipliziert werden.

Aus der deutschen Patentanmeldung DE102004042060A1 ist ein Verfahren zum automatischen Steuern der Röntgendosisleistung zum Herstellen eines Bildes bei der Computertomografie bekannt, bei dem die Röhrenspannung bei einem objektspezifisch vorbestimmten und abgespeicherten Röhrenstrom automatisch justiert wird.

Aus der deutschen Offenlegung DE102006046047A1 ist ein Verfahren zur kombinierten Knochenaufhärtungs- und Streustrahlungskorrektur für Objekte mit wenigstens 2 Materialkomponenten bekannt.

Aus der EP660599B2 ist ein experimentelles Verfahren zur Streustrahlenkorrektur bekannt, bei der von den logarithmierten Biilddaten ein experimentell ermitteltes Streustrahlenbild subtrahiert wird.

Aus der DE102005018660B4 ist ein Verfahren zur empirischen Bestimmung einer Cupping-Funktion zur Beseitigung von strahlungsaufhärtungs- und streustrahlenbedingten Effekten von Bilddaten eines flächenhaften Festkörper-Röntgendetektors bekannt.

Aus der DE102006049664A1 ist eine Kalibriermethode für Mehrspektrentomografie bekannt.

Aus der US5400387A ist bekannt, zur Korrektur der sensibel von der Beschleunigungsspannung der Röntgenröhre abhängigen Strahlungsaufhärtungseffekte die Röhrenspannung zu messen und die Aufhärtungskorrektur gemäß der gemessenen Röhrenspannung vorzunehmen.

Aus der DE68911072T2 ist eine Streustrahlenkorrektur bekannt, bei der eine von der Röntgenröhrenspannung und von Objekteigenschaften abhängige Streustrahlenmatrix von der ursprünglichen Bildmatrix subtrahiert wird.

Aus der DE102007046359A1 ist ein Verfahren für die Erstellung von materialselektiven Volumenbildern bekannt, bei dem ein Objekt mit einem C-Bogen-Röntgengerät aus unterschiedlichen Projektionsrichtungen und in unterschiedlichen Energiebereichen aufgenommen wird und die durch Rückprojektion der rekonstruierten Volumenbilder erhaltenen Projektionsbilder korrigiert werden (Absatz [0030]).

Aus der US5418373A ist ein Verfahren zur Knochendichtemessung bekannt, bei dem für wenigstens zwei Röhrenspannungen Röntgenbilder mit einem Speicherleuchtschirm aufgenommen und nach Auslesen, Logarithmieren und Digitalisieren die digitalen Bildsignale hinsichtlich der Strahlungsaufhärtungseffekte durch Subtraktion von Korrekturbildsignalen korrigiert werden.

Aus der DE102006045722A1 ist ein Verfahren zur Streustrahlenkorrektur bekannt, bei dem objektspezifische Faltungskerne auf die bei der Durchstrahlung dieser Objekte erzeugten Detektordaten angewendet werden.

Aus dem Zeitschriftenartikel "Empirical Cupping Correction" (M. Kachelrieß, K. Sourbelle, and W. Kalender, "Empirical cupping correction: A first order rawdata precorrection for cone-beam computed tomography," Med. Phys., vol. 33, no. 5, pp. 1269-1274, Mai 2006) ist ein Verfahren zur empirischen Korrektur der Strahlungsaufhärtung und der Streustrahlung in einem wasserähnlichen Objekt bekannt, bei dem polychromatische Röntgenbremsspektren mit gleicher Grenzenergie verwendet werden.

Aufgabe der Erfindung ist es, ein kostengünstiges und schnell arbeitendes Verfahren zur empirischen Korrektur der Strahlungsaufhärtung von polychromatischen Röntgenbremsspektren unterschiedlicher Grenzenergie an einem wasserähnlichen Objekt zu finden.

Die Aufgabe der Erfindung wird dadurch gelöst, daß eine von der Beschleunigungsspannung der Röntgenröhre bei einer Projektionsaufnahme abhängige Korrekturfunktion auf die aus den logarithmierten Meßwerten der Intensität gebildeten Projektionswerte qi angewandt wird und daß die Korrekturfunktion aus den Projektionsaufnahmen eines einzigen Scans mit variabler Beschleunigungsspannung an einem objektähnlichen Kalibrierphantom aus einem wasseräquivalenten Material gewonnen wird.

Die Erfindung wird anhand der nachstehenden theoretischen Zusammenhänge näher erläutert. Dabei wird lediglich der über die Herleitung des Korrekturfunktion für ein polychromatisches Röntgenspektrum mit einer bestimmten Grenzenergie aus dem Zeitschriftenartikel "Empirical Cupping Correction" (M. Kachelrieß, K. Sourbelle, and W. Kalender, "Empirical cupping correction: A first order rawdata precorrection for cone-beam computed tomography," Med. Phys., vol. 33, no. 5, pp. 1269-1274, Mai 2006) hinausgehende Teil der Theorie wiedergegeben.

Das physikalische Gesetz, auf dem die weiteren Betrachtungen aufbauen, ist Lambert-Beer's Gesetz, welches die Abschwächung eines polychromatischen Röntgenstrahls in einem Material mit den energie- und materialabhängigen Schwächungskoeffizienten (E, r) entlang des Weges d beschreibt. Der Einfachheit halber gehen wir im folgenden von homogenen Körpern aus einem Material mit konstanter Dichte aus, der Weg d kann also als Schnittlänge interpretiert werden:

$$I_a = \int dE \, w(E) \, I_0 e^{-\mu(E)d}$$

w(E) ist das auf 1 normierte Spektrum des emittierten Röntgenstrahls:

$$\int_0^{eU} dE \, w(E) = 1$$

$I_a$ ist die durch den Körper abgeschwächte Intensität, $I_0$ die Anfangsintensität des Röntgenstrahls, e die Elementarladung eines Elektrons und eU daher die maximal erreichbare Energie eines Röntgenphotons bei Röhrenspannung U.

Die tatsächlich gemessene Intensität im Detektor setzt sich zusammen aus $I_a$ und aus einem Streustrahlenanteil $I_s$. Dieser Streustrahlenanteil ist stark abhängig von der Geometrie und vom Material des zu vermessenden Körpers. Die Parameter, welche Is beeinflussen, werden im Folgenden als Gesamtheit mit dem Suffix beschrieben und an späterer Stelle näher spezifiziert.

Die Genauigkeit, mit der der Streustrahlanteil bestimmt wird (d.h. die Anzahl der Variablen von denen $I_s$ abhängt), erhöht die Genauigkeit der Vorkorrektur, da das Verhältnis von $I_a$ zu $I_s$ nicht genau bekannt ist.

$$I = I_a + I_s$$

Als polychromatischer Schwächungswert ist definiert

$$q(d,\alpha) = -\ln\frac{I(d,\alpha)}{I_0} = -\ln\frac{(I_A(d)+I_S(\alpha))}{I_0} = -\ln\frac{\left(I_A\left(1+\frac{I_S(\alpha)}{I_A(d)}\right)\right)}{I_0} =$$

$$-\ln\int dE\, w(E)\, e^{-\mu(E)d} - \ln\frac{1+\frac{I_S(\alpha)}{I_A(d)}}{I_0} = -\ln\int dE\, w(E)\, e^{-\mu(E)d} + \delta_S(d,\alpha)$$

s ist der Streustrahlenanteil im Raum der logarithmierten Rohdaten.

Bei polychromatischer Strahlung ist q eine in d nichtlineare Funktion. Ein linearer Zusammenhang ist nur bei einem monochromatischen Spektrum und ohne Streustrahlung gegeben:

$$w(E) = \delta(E - E_0)$$

(x) ist die Diracsche Deltafunktion.

Die Energie $E_0$ ist frei wählbar. Für Gleichung (2) gilt damit:

$$q_{E_0}(d) := p(d) = \mu(E_0)\, d$$

Da p und d sich nur um eine Proportionalitätskonstante unterscheiden, wird die Variable d im Folgenden nicht mehr mit aufgeführt. Dieser lineare Zusammenhang ist erforderlich, um aus Projektionsbildern korrekte Computertomografie-Bilder (CT-Bilder) zu rekonstruieren. Da monochromatische Spektren mit Röntgenröhren technisch nicht realisierbar sind und Streustrahlenanteile berücksichtigt werden sollten, müssen die polychromatischen Schwächungswerte entsprechend vorkorrigiert (linearisiert) werden. Benötigt wird also eine Korrekturfunktion p(q, ), die die polychromatischen und streustrahlenbehafteten Schwächungswerte auf monochromatische umrechnet. Als Referenzmaterial hierfür dient Wasser, daher bezeichnet man diese Methode auch als Wasservorkorrektur. Obige Betrachtungen gehen davon aus, daß das emittierte Spektrum w(E) für alle Projektionen eines Scans gleichbleibend konstant ist. Die bereits erwähnte Methode "ECC" ist ebenfalls für ein konstantes Spektrum vorgesehen.

Im Fall variabler Röhrenspannung U ist das Spektrum w(E,U) nicht mehr konstant sondern ist abhängig von der Röhrenspannung U abhängig. Dadurch erhält man für das durchstrahlte Objekt einen nicht nur vom Objektmaterial und der Objektdicke abhängigen sondern zusätzlich spektrums- oder spannungsabhängigen Schwächungswert q(p, U, ). Ebenso wird nun für jeden Wert der Röhrenspannung eine separate Korrekturfunktion p(q, ,U) benötigt.

Eine Methode, dies zu tun, ist aus der deutschen Offenlegungsschrift DE102005028216A1 beschrieben. Dort ist vorgesehen, die Funktion p(q,U) in Form diskreter Werte in einem Speicher zu hinterlegen. Die unkorrigierten Werte q werden mit den entsprechenden Werten aus dem Speicher korrigiert.

Die nachfolgend beschriebene Methode ermöglicht eine einfache Bestimmung der Korrekturfunktion p(q,U, ) unter Berücksichtigung der Streustrahleneigenschaften des Kalibrierkörpers, wobei die erhaltene Korrekturfunktion mit geringem Rechenaufwand auf die logarithmierten Meßwerte der Pixelwerte des Rohbildes angewandt werden.

Als Lösungsansatz für die unbekannten Funktion p(q,U, ) geht man von einer Linearkombination von Basisfunktionen, vorzugsweise von Polynomen, aus. Die als Polynom angesetzte Korrekturfunktion weist nicht das gleiche Monotonieverhalten auf wie die unbekannte Funktion p(q,U, ). Durch die Wahl anderer Basisfunktionen, die dem zugrundeliegenden physikalischen Modell besser entsprechen, kann dies verhindert werden, so daß auch eine korrekte Extrapolation der Funktion in nicht durch Meßwerte erfaßte aber relevante Wertebereiche möglich ist.

Als mögliche Basisfunktionen kommen diejenigen in Betracht, die in p(q,U) folgende Eigenschaften aufweisen:

- p(q,U) steigt mit U monoton an
- p(q,U) steigt mit q
- Die erste Ableitung von p(q,U) nach q ist positiv, monoton steigend und konvergiert im Unendlichen gegen den Wert (E0)/ (eU), da dann gilt q= (eU)*d (nach unendlicher Materialdicke ist der Strahl theoretisch monochromatisch, es bleibt in Wirklichkeit aber keine meßbare Intensität übrig) und p= (E0)*d per Definition, wobei eU die maximale Energie der Röhre ist, und E0 ein frei wählbarer Energiewert ist, auf den kalibriert wird.

Um dem korrekten Monotonieverhalten der gesuchten Funktion p(q,U, ) gerecht zu werden ist vorgesehen, die Polynomfunktionen nach ihrer Bestimmung mit einer geeigneteren Funktion zu ersetzen. Folgender Ansatz erfüllt die oben genannten Kriterien:

$$p(U,\alpha,q) = \frac{c_0(U,\alpha) + c_1(U,\alpha)q + ... + c_n(U,\alpha)q^n}{1 + d_1(U,\alpha)q + ... + d_{n-1}(U,\alpha)q^{n-1}}$$

Zusätzlich muß gelten: $\dfrac{c_n}{d_{n-1}} = \dfrac{\mu(E_0)}{\mu(eU)}$ damit die Funktion gegen den $d_{n-1}$ $\mu(eU)$ korrekten Wert konvergiert.

Diese Funktion kann nicht direkt als Basisfunktion genutzt werden, da sie als gebrochen rationale Funktion keine Linearkombination darstellt. Stattdessen wird nun für jeden Wert U und die gebrochen rationale Funktion an das vorher ermittelte Polynom im gemessenen Wertebereich angefittet, da dort die Korrektheit der Polynomfunktion sicher ist. Durch die eingeführte Randbedingung und das sonstige Verhalten der gebrochen rationalen Funktion ist auch ein korrektes Verhalten außerhalb der gemessenen Werte gegeben.

Die Korrektur von Aufnahmen eines späteren Scans kann mittels einer Polynomfunktion erfolgen, sofern die Werte für die Grenzspannung des Röntgenbremsspektrums bei der jeweiligen Aufnahme innerhalb des bei der Kalibrierung mit einem Wasserphantom ähnlicher Geometrie verwendeten Bereiches von Grenzspannungen liegen. Für alle Fälle, in denen bei einem Scan die Grenzspannung bei einer Aufnahme außerhalb des bei der Kalibrierung verwendeten Bereiches von Grenzspannungen liegt, erfolgt die Korrektur der Strahlungsaufhärtungs- und Streustrahleneffekte mit der an die Polynomfunktion angefittete gebrochen rationale Funktion.

Die Korrekturfunktion p(q,U) wird aus Projektionsaufnahmen eines Scans mit einem flächigen, bildgebenden Detektor bei Kegel- oder Pyramidenstrahlgeometrie des Röntgenstrahlenbündels ermittelt.

Für die Kalibrierung werden unterschiedlich große zylindrische Körper (Kalibrierphantome) aus wasserähnlichem Material und elliptischer Grundfläche verwendet, die jeweils durch den objektabhängigen Parameter O gekennzeichnet sind. Das heißt, man erhält eine Schar von Korrekturfunktionen $p_O$ (q, U) jeweils durch einen einzelnen Scan am Kalibrierkörper O. Dies impliziert die Annahme, daß der unbekannte Streuanteil auch nur von den Variablen q, U und O abhängt.

Berücksichtigt wird lediglich die durch wasserähnliches Material verursachte Streustrahlung; stark streuende Strukturen im Inneren des Objekts, wie kontrastmittelgefüllte Gefäße, Knochen oder Metallteile bleiben unberücksichtigt.

Für die Kalibrierung werden oben beschriebene elliptische Wasserzylinder mit maximaler Höhe genutzt, deren Zylinderachse derart ausgerichtet ist, daß sie senkrecht auf dem Zentralstrahl der Röntgenaufnahmeeinheit steht. In der Praxis hat sich gezeigt, daß mit vier verschieden großen Kalibrierphantomen alle Funktionen zur Korrektur der Strahlungsaufhärtungs- und Streustrahleneffekte mit hinreichender Genauigkeit bestimmen lassen. Dabei ist das bei den Kalibrierscans verwendete größte Kalibrierphantom so zu wählen, daß es bei bestimmten Detektorabmessungen und einem bestimmten Fokus-Detektor-Abstand in Scanrichtung vollständig abgebildet wird. Es hat sich gezeigt, daß die Wasser-Vorkorrektur um so genauer ist, je ähnlicher das Kalibrierphantom dem durchstrahlten Untersuchungsobjekt ist.

Genauere geometrische Details wie z.B. Kanten, an denen Streuung auch besonders zu beobachten ist, werden bei der Bestimmung der Korrekturfunktion vernachlässigt; daher handelt es sich hierbei auch nur um eine Näherung zur Korrektur des Streustrahlenanteils.

Im Weiteren wird der Parameter O der Einfachheit halber vernachlässigt, da derselbe Kalibriervorgang für verschiedene Objekte O der gleiche ist.

$$p(q,U) = \sum_{k,l} c_{k,l} q^k U^l$$

Die gesuchte Funktion wird in diesem Fall vollständig durch die Koeffizienten $c_{k,1}$ bestimmt.

Die Rückprojektion der Projektionsdaten in ein Volumen zur Erzeugung von CT-Bildern erfolgt über die inverse Radontransformation $R^{-1}$.

$$f(\mathbf{r}) = \mathbf{R}^{-1} p(q,U) = \sum_{k,l=0}^{K,L} \mathbf{R}^{-1}\left(c_{k,l} q^k U^l\right) = \sum_{k,l=0}^{K,L} c_{k,l} f_{k,l} = \sum_{n=1}^{N=(K+1)(L+1)} c_n f_n$$

, mit $n = k \cdot L + l$.

Der Vektor r bezieht sich auf das Volumen, in das die Daten rückprojiziert werden. Da das Volumen diskretisiert ist, stellt r einen Voxel dar. Die Tatsache, daß die Rückprojektion linear bezüglich der Basisfunktionen ist, macht es möglich, die Basisfunktionen einzeln zu rekonstruieren ($f_n$) und die Linearkombination im Bildraum auszuführen. Da man weiß, welche Eigenschaften das ideale rekonstruierte Bild hat, nämlich konstante Grauwerte für Bereiche konstanter Dichte und gleichen Materials, kann man die Linearkombination dahingehend optimieren. Aus dem artefaktbehafteten Bild läßt sich ein Templateimage t(r) konstruieren, indem die Bereiche Luft und Wasser getrennt und auf die gewünschten konstanten Grauwerte gesetzt werden.

Das aus korrigierten Daten rekonstruierte Bild f(r) soll folgende Bedingung erfüllen:

$$E^2 = \sum_{\mathbf{r}} w(\mathbf{r})\left(f(\mathbf{r}) - t(\mathbf{r})\right)^2 = \min$$

w(r) ist das sogenannte Gewichtungsbild mit den Werten 0 und 1, das Bereiche außerhalb des Meßfeldes sowie Kantenübergänge (Punktbildfunktionsungenauigkeiten) und Bereiche in denen keine sichere Materialdetektion erfolgte von der Koeffizientenbestimmung ausschließt. Um den optimalen Satz an Koeffizienten zu finden, der obige Bedingung erfüllt, leitet man $E^2$ nach $c_n$ ab. Dies führt zum linearen Gleichungssystem $a = B \cdot c$, mit

$$a_n = \sum_{\mathbf{r}} w(\mathbf{r}) t(\mathbf{r}) f_n(\mathbf{r})$$

$$B_{nm} = \sum_{\mathbf{r}} w(\mathbf{r}) f_n(\mathbf{r}) f_m(\mathbf{r})$$

Durch Invertieren von B läßt sich der gesuchte Koeffizientenvektor c bestimmen, da

$$c = B^{-1} \cdot a.$$

Vorteilhaft ist, daß beim Kalibrierscan eine möglichst große Bandbreite an Grenzspannungen der Röntgenbremsspektren (Röhrenspannungen) und an Strahldurchdringungslängen vorliegt. Das Problem wird durch die Ähnlichkeit des Kalibrierphantoms mit dem Untersuchungsobjekt gelöst abgedeckt wird, so daß die bei einem späteren Scan auftretenden Spannungen und Schwächungswerte in der Kalibrierung berücksichtigt sind.

Zu jedem Pixelwert qi wird die Funktion pi(qi, U) berechnet, wobei als Spannung die eingeregelte Röhrenspannung herangezogen wird, mit der das Röntgenprojektionsbild aufgenommen wurde. In der Praxis ist es so, daß die Dosisleistungsregelung die Röhrenspannung in einem Bruchteil der Belichtungszeit einregelt, so daß die Spannungsvariation während des Einregelns nicht berücksichtigt zu werden braucht.

Nach der Beendigung einer Projektionsaufnahme werden die einzelnen Pixelwerte, welche die gemessene Intensität der Röntgenquanten repräsentieren, aus dem bildgebenden Detektor ausgelesen, logarithmiert und in einem Speicher als Rohbild abgelegt. Laut Definition ist

$$q(p, U, O) = -\ln \frac{I(p, U, O)}{I_0} = -\ln I(p, U, O) + \ln I_0$$

Das heißt alle mit qi bezeichneten Pixelwerte sind bereits logarithmiert. Da der Wert $I_0$ vor einer Kalibrierung nicht bekannt ist, macht es Sinn den Offset $\ln(I_0)$ in die Korrekturfunktion mit aufzunehmen (entspricht dann dem q°-Teil im Polynom, da dies ein nur von U abhängiger Wert ist, der aufaddiert wird ), in diesem Fall ist

$$q = -\ln I$$

der bezüglich Strahlaufhärtung, Streustrahlung und $I_0$ Wert unkorrigierte logarithmierte Meßwert.

Zu jedem Wert qi des Rohbildes wird die Funktion pi(qi, U) berechnet, wobei als Spannung die eingeregelte Röhrenspannung herangezogen wird, mit der das Röntgenprojektionsbild aufgenommen wurde. In der Praxis ist es so, daß die Dosisleistungsregelung die Röhrenspannung in einem Bruchteil der Belichtungszeit einregelt, so daß die Spannungsvariation während des Einregelns nicht berücksichtigt zu werden braucht.

Aus der Schar der Korrekturfunktionen wird diejenige ausgewählt, die mit einem Kalibrierphantom experimentell ermittelt wurde, welches dem Untersuchungsobjekt in Form und Dicke möglichst nahe kommt. Die Auswahl der objektabhängigen Korrekturfunktion kann aufgrund weiterer Messungen am Objekt automatisch oder manuell durch eine entsprechende Eingabeprozeduren erfolgen.

Das Anwenden der Korrekturfunktion $p_o,(q, U)$ auf jeden einzelnen Pixelwert qi ergibt ein vorkorrigiertes Bild mit den Werten pi, die die korrigierten, logarithmierten Meßwerte darstellen.

Nach dieser Vorkorrektur wird das vorkorrigierte Bild entsprechend dem Zweck der Aufnahme weiter verarbeitet, beispielsweise zusammen mit weiteren vorkorrigierten Röntgenprojektionen für eine 3D-Rekonstruktion des Objekts, zur Durchführung von Knochendichtemessungen oder zur Durchführung von Verfahren zur Digitalen Subtraktionsangiografie (DSA).

Das Rechenverfahren zur Vorkorrektur wird nach Beendigung des Aufnahmevorganges einer jeden Projektionsaufnahmen für diese Projektionsaufnahme begonnen und ist in der Regel zum Zeitpunkt der Beendigung der nachfolgenden Projektionsaufnahme beendet, so daß die korrigierte Projektionsaufnahme in Echtzeit dargestellt oder/und weiter verarbeitet werden kann.

Der Kalibrierscan unter Verwendung der Röntgendiagnostikeinrichtung und eines Kalibrierphantoms kann auf zweierlei Weise aufgenommen werden:

1. Wenn die Röntgendiagnostikeinrichtung über eine Möglichkeit verfügt, um ein Untersuchungsobjekt herum verschwenkt zu werden, beispielsweise, wenn die Röntgenaufnahmeeinheit an einem mehrfach verstellbaren C-Bogen oder in einem, cone-beam-Computertomografen angeordnet ist, kann das Kalibrierphantom raumfest angeordnet sein und die Projektionsaufnahmen können durch Verstellung der Röntgenaufnahmeeinheit gewonnen werden.

2. Das Kalibrierphantom kann bei einer starren, bei einer verstellbaren aber nicht für einen Scan geeigneten oder bei einer zur Aufnahme eines Scans geeignete verstellbaren Röntgenaufnahmeeinheit derart angeordnet werden, daß es innerhalb des Strahlenkegels der Röntgenaufnahmeeinheit zu liegen kommt und bei raumfester Röntgenaufnahmeeinheit um eine senkrecht zur durch den Fokus der Röntgenstrahlenquelle und dem Mittelpunkt des bildgebenden Röntgendetektors verlaufenden Zentralstrahls und parallel zu der Zylinderachse verlaufenden Drehachse schrittweise verstellt wird und bei jedem Winkelschritt eine zugehörige Röntgenprojektionsaufnahme des Kalibrierphantoms aufgenommen wird.

Der Kalibrierscan besteht aus einzelnen Projektionsaufnahmen eines Kalibrierphantoms, die mit unterschiedlichen Beschleunigungsspannungen der Röntgenröhre aufgenommen wurden.

Die Variation der Beschleunigungsspannung während des Kalibrierscans kann durch eine automatische Verstärkungs- und Dosisleistungsregelung der Röntgendiagnostikeinrichtung derart automatisch erfolgen, daß die erzeugten Projektionsaufnahmen bei hinreichender Helligkeit einen hinreichend guten Kontrast aufweisen.

Es ist im Rahmen der Erfindung vorgesehen, die Beschleunigungsspannung während des Kalibrierscans mittels einer Steuerung derart zu steuern, daß zwischen einer unteren und einer oberen Beschleunigungsspannung diskrete, äquidistante Zwischenwerte mit gleicher Häufigkeit zur Aufnahme der Projektionsaufnahmen des Kalibrierphantoms verwendet werden.

Durch die Verwendung eines in seiner Drehlage bezüglich des Zentralstrahls verstellbaren Kalibrierphantoms kann jede Röntgendiagnostikeinrichtung hinsichtlich der empirischen Wasserkorrektur korrigiert werden.

Die Korrekturfunktion p(q,U) wird als Vorkorrektur auf jeden Schwächungswert q eines Pixel einer Projektionsaufnahme eines Untersuchungsobjektes ähnlicher Abmessungen wie das Kalibrierphantom angewandt, ungeachtet der Tatsache, daß das Untersuchungsobjekt nicht ausschließlich aus einem wasserähnlichen Material besteht.

In der Praxis werden bei der Durchführung der Vorkorrektur diejenigen look-up-tables (LUT) von Koeffizienten von Korrekturfunktionen ausgewählt, die einem Kalibrierphantom zugeordnet sind, welches dem Untersuchungsobjekt am ähnlichsten ist. Liegt die bei der Projektionsaufnahme an die Röntgenröhre angelegte Beschleunigungsspannung innerhalb des beim Kalibrierscan abgedeckten Bereiches von Beschleunigungsspannungen, so wird die Polynom-Korrekturfunktion angewendet; liegt die bei der Projektionsaufnahme an die Röntgenröhre angelegte Beschleunigungsspannung außerhalb des beim Kalibrierscan abgedeckten Bereiches von Beschleunigungsspannungen, so wird die an die Polynom-Korrekturfunktion angefittete gebrochen rationale Korrekturfunktion angewendet.

Verzeichnis der Abbildungen:

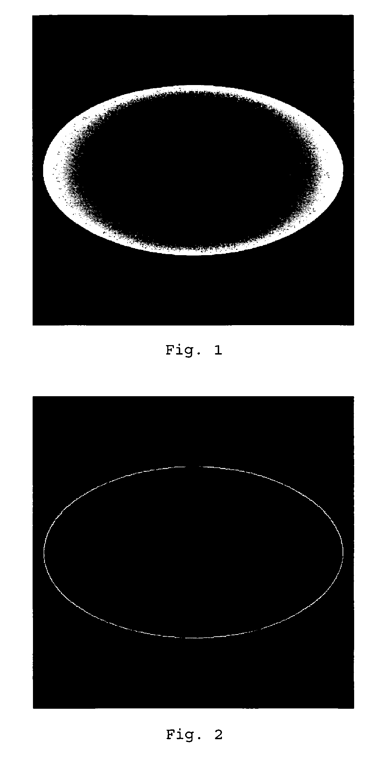

Fig. 1: CT-Bild eines Wasserphantoms aus unkorrigierten Projektionswerten q bestimmt (Simulation). Die Röhrenspannung variierte zwischen 40 kV (kurze Halbachse) und 110 kV (lange Halbachse).

Fig. 2: Aus unkorrigierten Projektionswerten erzeugtes Templateimage t(r). Grauwerte: Luft= 1000 HU, Wasser= 0 HU (entspricht Hounsfieldeinheiten).

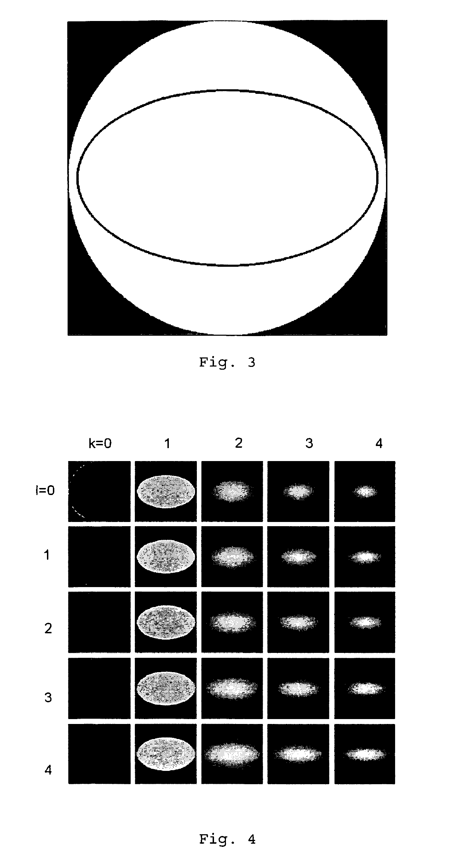

Fig. 3: Aus unkorrigierten Projektionswerten erzeugtes Gewichtungsbild w(r). Die weißen Bereiche haben den Wert 1, die grauen den Wert 0.

Fig. 4: Basisbilder $f_{k,1}(r) = f_n(r)$

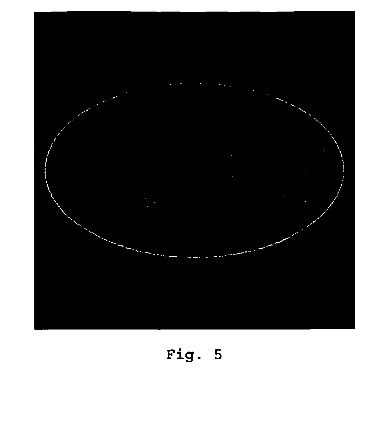

Fig. 5: Korrigiertes Bild f(r)

## Patentansprüche

1. Verfahren zur empirischen Bestimmung einer Korrekturfunktion zur Korrektur von Strahlungsaufhärtungs- und Streustrahleneffekten von wasseräquivalentem Gewebe eines Untersuchungsobjektes in der Projektionsradiografie und in der Computertomografie unter Verwendung einer Röntgenaufnahmeeinheit aus einer polychromatischen Röntgenstrahlenquelle variabler Beschleunigungsspannung U, einem bildgebenden, digitalen Röntgendetektor und einem Bildverarbeitungsrechner, wenigstens eines im Bereich des Röntgenstrahlenkegels zwischen der Röntgenstrahlenquelle und dem Röntgendetektor angeordneten, zylindrischen Kalibrierphantoms aus wasserähnlichem Material mit elliptischer Grundfläche, dessen Zylinderachse senkrecht auf dem zwischen dem Fokus der Röntgenstrahlenquelle und dem Mittelpunkt des Röntgendetektors verlaufenden Zentralstrahl steht und das der Form des Untersuchungsobjekts im Bereich des Strahlenkegels ähnlich ist, **gekennzeichnet durch folgende Verfahrensschritte:**

a) Von dem wenigstens einen Kalibrierphantom werden unter einer Vielzahl von unterschiedlichen Winkeln zwischen der Hauptachse der Ellipse und dem Zentralstrahl der Röntgenaufnahmeeinheit in Form eines Kalibrierscans Röntgenprojektionsbilder des Kalibrierphantoms mit unterschiedlichen Beschleunigungsspannungen aufgenommen und im Speicher des Bildverarbeitungsrechners abgespeichert

b) Aus den Projektionsaufnahmen des Kalibrierscans wird das Volumen des Kalibrierphantoms im Voxel mit dem Vektor r über eine inverse Radontransformation $R^1$ mit der Bedingung $n = k \cdot L + l$ rekonstruiert:

$$f(\mathbf{r})=\mathbf{R}^{-1}p(q,U)=\sum_{k,l=0}^{K,L}\mathbf{R}^{-1}\left(c_{k,l}q^{k}U^{l}\right)=\sum_{k,l=0}^{K,L}c_{k,l}f_{k,l}=\sum_{n=1}^{N=(K+1)(L+1)}c_{n}f_{n}$$

Dem artefaktbehafteten rekonstruierten Bild wird ein Templatebild t(r) zugeordnet, bei dem die Bereiche mit Luft und Wasser getrennt und auf vordefinierte, konstante Grauwerte gesetzt werden, wobei das aus den korrigierten Daten rekonstruierte Bild f(r) die folgende Bedingung erfüllt:

$$E^{2}=\sum_{\mathbf{r}}w(\mathbf{r})\left(f(\mathbf{r})-t(\mathbf{r})\right)^{2}=\min$$

, wobei w(r) ein Gewichtungsbild mit den Werten 0 und 1 ist. Die Gleichung für $E^2$ wird nach $c_n$ abgeleitet, woraus sich ein lineares Gleichungssystem $a = B \cdot c$, mit

$$a_{n}=\sum_{\mathbf{r}}w(\mathbf{r})t(\mathbf{r})f_{n}(\mathbf{r})$$

$$B_{nm}=\sum_{\mathbf{r}}w(\mathbf{r})f_{n}(\mathbf{r})f_{m}(\mathbf{r})$$

ergibt und **durch** Invertieren von B der gesuchte Koeffizientenvektor c gemäß

$$c = B^{-1} \cdot a.$$

und damit die gesuchte Polynom-Korrekturfunktion

$$p(q,U)=\sum_{k,l}c_{k,l}q^{k}U^{l}$$

bestimmt wird. Die für einen Bereich von Beschleunigungsspannungen der Röntgenröhre ermittelte Polynom-Korrekturfunktion wird in einer dem Kalibrierphantom zugeordneten Nachschlagetabelle (look-up-table, LUT) abgespeichert.
c) an die Polynom-Korrekturfunktion aus Verfahrensschritt b) wird im Bereich der beim Scan verwendeten Beschleunigungsspannungen für das der Funktion zugrunde liegende Kalibrierphantom α eine gebrochen rationale Funktion

$$p(U,\alpha,q)=\frac{c_{0}(U,\alpha)+c_{1}(U,\alpha)q+...+c_{n}(U,\alpha)q^{n}}{1+d_{1}(U,\alpha)q+...+d_{n-1}(U,\alpha)q^{n-1}}$$

mit der Nebenbedingung

$$\frac{c_n}{d_{n-1}} = \frac{\mu(E_0)}{\mu(eU)}$$

angefittet.

Die Koeffizienten der gebrochen rationalen Funktion werden in einer dem Kalibrierphantom $\alpha$ zugeordneten Nachschlagetabelle (look-up-table, LUT) abgespeichert.

d) Die Verfahrensschritte a) bis c) werden für weitere Kalibrierphantome unterschiedlicher Geometrie wiederholt, bis eine hinreichende Anzahl von LUT für unterschiedliche Kalibrierphantome bestimmt wurde.

2. Verfahren zur empirischen Bestimmung einer Korrekturfunktion nach Anspruch 1, **dadurch gekennzeichnet, daß** bei dem Verfahrensschritt a) die Beschleunigungsspannung während des Kalibrierscans durch eine automatische Verstärkungs- und Dosisleistungsregelung der Röntgendiagnostikeinrichtung derart variiert wird, daß die erzeugten Projektionsaufnahmen bei hinreichender Helligkeit einen hinreichend guten Kontrast aufweisen.

3. Verfahren zur empirischen Bestimmung einer Korrekturfunktion nach Anspruch 1, **dadurch gekennzeichnet, daß** bei dem Verfahrensschritt a) die Beschleunigungsspannung während des Kalibrierscans von einer Steuerung derart gesteuert wird, daß zwischen einer unteren und einer oberen Beschleunigungsspannung diskrete, äquidistante Zwischenwerte mit gleicher Häufigkeit zur Aufnahme der Projektionsaufnahmen des Kalibrierphantoms verwendet werden.

4. Verfahren zur Korrektur von Strahlungsaufhärtungs- und Streustrahleneffekten von wasserähnlichen Materialien in Röntgenprojektionsaufnahmen eines Untersuchungsobjektes unter Verwendung von nach den Ansprüchen 1-3 ermittelten Korrekturfunktionen, **gekennzeichnet durch folgende Verfahrensschritte:**

a) Auswahl der LUT von Koeffizienten von Korrekturfunktionen, die einem Kalibrierphantom zugeordnet sind, welches dem Untersuchungsobjekt am ähnlichsten ist,
b) Anwendung der Polynom-Korrekturfunktion auf den logarithmierten Schwächungswert q jedes Pixels, wenn die bei der Projektionsaufnahme an die Röntgenröhre angelegte Beschleunigungsspannung innerhalb des beim Kalibrierscan abgedeckten Bereiches von Beschleunigungsspannungen liegt und Anwendung der gebrochen rationalen Korrekturfunktion auf den logarithmierten Schwächungswert q jedes Pixels der Projektionsaufnahme, wenn die bei der Projektionsaufnahme an die Röntgenröhre angelegte Beschleunigungsspannung außerhalb des beim Kalibrierscan abgedeckten Bereiches von Beschleunigungsspannungen liegt.

Fig. 1

Fig. 2

Fig. 3

Fig. 4

Fig. 5

|  | Europäisches Patentamt<br>European Patent Office<br>Office européen des brevets | **EUROPÄISCHER RECHERCHENBERICHT** | **Nummer der Anmeldung**<br>EP 10 01 4671 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | LUDWIG RITSCHL ET AL: "Empirical cupping correction for CT scanners with tube voltage modulation (ECCU)",<br>2009 IEEE NUCLEAR SCIENCE SYMPOSIUM AND MEDICAL IMAGING CONFERENCE (NSS/MIC 2009), IEEE, ORLANDO, FL, USA,<br>24. Oktober 2009 (2009-10-24), Seiten 3950-9394, XP031621334,<br>ISBN: 978-1-4244-3961-4<br>* das ganze Dokument *<br>-----  | 1-4 | INV.<br>G06T11/00 |
|  |  |  | RECHERCHIERTE SACHGEBIETE (IPC)<br><br>G06T |

Der vorliegende Recherchenbericht wurde für alle Patentansprüche erstellt

| Recherchenort | Abschlußdatum der Recherche | Prüfer |
|---|---|---|
| München | 17. Februar 2011 | Werling, Alexander |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument
.................................................................
& : Mitglied der gleichen Patentfamilie, übereinstimmendes
   Dokument

EPO FORM 1503 03.82 (P04C03)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102005028216 A1
- DE 102005053498 B4
- DE 102006040852 A1
- DE 102006021373 A1
- DE 19523090 C1
- DE 102004042060 A1
- DE 102006046047 A1
- EP 660599 B2
- DE 102005018660 B4
- DE 102006049664 A1
- US 5400387 A
- DE 68911072 T2
- DE 102007046359 A1
- US 5418373 A
- DE 102006045722 A1

**In der Beschreibung aufgeführte Nicht-Patentliteratur**

- **M. KACHELRIEß ; K. SOURBELLE ; W. KALEND-ER.** Empirical cupping correction: A first order rawdata precorrection for cone-beam computed tomography. *Med. Phys.,* Mai 2006, vol. 33 (5), 1269-1274